## Europäisches Patentamt
## European Patent Office
## Office européen des brevets

(19)

(11) Veröffentlichungsnummer: **0 054 152**
. B1

(12)

# EUROPÄISCHE PATENTSCHRIFT

(45) Veröffentlichungstag der Patentschrift:
27.02.85

(21) Anmeldenummer: 81108855.8

(22) Anmeldetag: 24.10.81

(51) Int. Cl.⁴: **C 07 C 69/24,** C 07 C 67/38,
C 07 C 29/136, C 07 C 29/76

(54) Verfahren zur Herstellung von weitgehend von stickstoffhaltigen Verbindungen befreiten Folgeprodukten aus durch Hydrocarboxilierung erhaltenen Carbonsäurealkylestern.

(30) Priorität: 11.12.80 DE 3046651

(43) Veröffentlichungstag der Anmeldung:
23.06.82 Patentblatt 82/25

(45) Bekanntmachung des Hinweises auf die Patenterteilung:
27.02.85 Patentblatt 85/9

(84) Benannte Vertragsstaaten:
AT BE FR GB IT NL

(56) Entgegenhaltungen:
FR - A - 1 550 593

(73) Patentinhaber: CHEMISCHE WERKE HÜLS AG,
Postfach 1320, D-4370 Marl 1 (DE)

(72) Erfinder: Hofmann, Peter, Dr., Lipper Weg 193,
D-4370 Marl (DE)
Erfinder: Müller, Wolfgang Hans Eduard, Dr., Bitterfelder
Strasse 9 a, D-4370 Marl (DE)

## Beschreibung

Es ist bekannt, dass man durch Umsetzung von Olefinen mit Kohlenmonoxid und Alkanolen in Gegenwart eines Katalysators, der ein Metall der 8. Nebengruppe des Periodischen Systems der Elemente und ggf. einen Promotor enthält, Fettsäureester herstellen kann (J. Falbe, ,,Synthesen mit Kohlenmonoxid", Springer-Verlag, Berlin, Heidelberg, New York [1967]).

Als bevorzugte Variante dieser als Hydrocarboxilierung bezeichneten Reaktion hat sich die Umsetzung in Gegenwart von cobalthaltigen Katalysatoren erwiesen. Eine besonders bevorzugte Ausführungsform besteht darin, dass man zusätzlich einen Promotor, und zwar insbesondere Pyridin oder ein nichtorthosubstituiertes Alkylpyridin zusetzt.

Die durch Hydrocarboxilierung erhaltenen Carbonsäurealkylester sind vielseitig verwendbare Zwischenprodukte, die u.a. zu Alkoholen weiterverarbeitet werden können. Die auf diesem Wege hergestellten Alkohole sind jedoch — bedingt durch die Verwendung stickstoffhaltiger Promotoren (Pyridin und/oder nichtorthosubstituierte Alkylpyridine) auf der Hydrocarboxilierungsstufe — stets mit geringen Mengen stickstoffhaltiger Verbindungen verunreinigt. Handelsübliche, z.B. durch Hydroformylierung, Ziegler-Aufbau-Reaktion, Paraffinoxidation oder durch Fettspaltung hergestellte Alkohole enthalten in der Regel solche Verunreinigungen nicht. Es ist daher angebracht, auch die Folgeprodukte der durch Hydrocarboxilierung erhaltenen Carbonsäurealkylester, vorzugsweise Alkohole, so herzustellen, dass sie frei bzw. weitgehend frei von stickstoffhaltigen Verunreinigungen sind.

Durch Destillation der Alkohole bzw. der als Vorstufe verwendeten Carbonsäureester ist es nicht möglich, die stickstoffhaltigen Verunreinigungen ausreichend zu eliminieren. Selbst bei Verwendung von Destillationskolonnen hoher Bodenzahlen und bei Einhaltung hoher Rücklaufverhältnisse beobachtet man nur eine in etwa gleichmässige Verteilung des Stickstoffgehaltes über alle Destillatfraktionen.

Auch durch die Behandlung der Alkohole bzw. Carbonsäureester mit Adsorptionsmitteln, wie z.B. Aktivkohle, Aluminiumoxid verschiedener Aktivitätsstufen oder Kieselsäure, lässt sich eine nur unzureichende Entfernung der stickstoffhaltigen Verunreinigungen erzielen.

Schliesslich lassen sich durch die Behandlung der Carbonsäureester mit sauren Ionenaustauschern die stickstoffhaltigen Verunreinigungen nur unzureichend entfernen.

Aufgabe der vorliegenden Erfindung war es daher, ein technisch einfaches und wirtschaftliches Verfahren zur Herstellung von weitgehend von stickstoffhaltigen Verbindungen befreiten Folgeprodukten, vorzugsweise Alkoholen, aus durch Hydrocarboxilierung erhaltenen Carbonsäurealkylestern zu entwickeln.

Diese Aufgabe wurde überraschenderweise durch die in den Ansprüchen beschriebenen Massnahmen gelöst.

Überraschenderweise deshalb, weil es einmal nicht zu erwarten war, dass unter den für die Esterhydrierung typischen, relativ milden Bedingungen die aus dem Promotor stammenden stickstoffhaltigen Verbindungen in eine solche Form überführt werden, dass sie durch eine anschliessende Behandlung mit einem sauren Ionenaustauscher vollständig bzw. weitgehend entfernt werden können (vgl. F. Zymalkowsky, ,,Katalytische Hydrierung", F. Enke Verlag, Stuttgart [1965], Kap. XI, S. 118).

Zum anderen, weil die Wirksamkeit des zur Esterhydrierung benutzten Katalysators trotz der Anwesenheit von üblicherweise als Kontaktgifte geltenden Stickstoffverbindungen nicht beeinträchtigt wird (vgl. F. Zymalkowsky, ,,Katalytische Hydrierung", F. Enke Verlag, Stuttgart [1965], Kap. IV, S. 34; ,,Organic Reactions", John Wiley & Sons, Inc., New York [1954], vol. VIII, S. 11).

Die beim erfindungsgemässen Verfahren eingesetzten Carbonsäurealkylester können durch alle Hydrocarboxilierungsreaktionen erhalten werden, bei denen Olefine mit Kohlenmonoxid und Alkanol in Gegenwart eines aus einer Cobaltverbindung und Pyridin und/oder nichtorthosubstituierten bestehenden Katalysatorsystems bei erhöhtem Druck und erhöhter Temperatur umgesetzt werden (z.B. Verfahren gemäss US-PS Nr. 3507891 und DE-OS Nr. 2912489). Dabei ist die Wahl des eingesetzten Olefins unkritisch, d.h. es können sowohl geradkettige oder verzweigte $\alpha$-Olefine als auch Olefine mit innenständiger Doppelbindung eingesetzt werden. Aber auch Olefine mit mehr als einer Doppelbindung und solche mit Substituenten, wie z.B. Aryl-, Carboximethyl- und Hydroxylgruppen, sind geeignet.

Im allgemeinen werden Olefine mit 2 bis 40, vorzugsweise mit 4 bis 20 Kohlenstoffatomen eingesetzt, die nach bekannten Verfahren des Standes der Technik erhalten werden können. So können z.B. $\alpha$-Olefine durch die Aufbaureaktion von Ethylen nach Ziegler oder durch Wachscracken, Olefine mit innenständiger Doppelbindung, die bevorzugt beim erfindungsgemässen Verfahren eingesetzt werden, durch katalytische Dehydrierung von Paraffinen oder durch Chlorierung von Paraffinen und anschliessender Dehydrochlorierung der Chlorparaffine gewonnen werden (GB-PS Nr. 1037868).

Bei dem zuletzt genannten Verfahren werden in der Regel Paraffinschnitte, d.h. Gemische unterschiedlicher C-Zahl eingesetzt, so dass auch die erhaltenen Olefine keine einheitliche C-Zahl aufweisen. Ausserdem kommen in diesen Olefingemischen natürlich alle denkbaren isomeren Formen vor.

Neben den reinen — ggf. substituierten — Olefinen können auch solche mit einem Gehalt an Paraffinen, z.B. bis zu 85 Gew.-%, eingesetzt werden. Der Paraffingehalt rührt daher, dass bei der Olefinherstellung keine vollständigen Umsätze erreicht und die nicht umgesetzten Paraffine nicht oder nicht vollständig abgetrennt werden.

Nicht nur das eingesetzte Olefin, sondern auch die Art des Alkanols, das mit dem Olefin und Kohlenmonoxid umgesetzt wird, ist für die Herstellung der bei dem erfindungsgemässen Verfahren eingesetzten Carbonsäurealkylester unkritisch. Im allgemeinen werden Alkanole mit 1 bis 10, vorzugsweise 1 bis 4 Kohlenstoffatomen eingesetzt. Typische Vertreter aus der Gruppe der primären Alkanole sind z.B. Methanol, Ethanol, Propanol-(1) und Butanol-(1).

Weiterhin ist es unwesentlich, welche Cobaltverbindung bei der Hydrocarboxilierung verwendet wird. Carbonyle des Cobalts, z.B. Dicobaltoctacarbonyl, sind ebenso geeignet wie carbonsaure Cobaltsalze, wie z.B. Cobaltacetat, Cobaltnaphthenat und Cobalt-2-ethylhexanoat, und Salze des Cobalts mit anorganischen Säuren, wie z.B. Cobaltnitrat und Cobaltsulfat. Vorzugsweise kommen carbonsaure Cobaltsalze zum Einsatz, deren Anionen dem Säurerest der bei der Hydrocarboxilierung gebildeten Fettsäureester entsprechen.

Als Promotoren kommen Pyridin und alle nichtorthosubstituierten Alkylpyridine, wie z.B. 3- und 4-Picolin, 3,4- und 3,5-Lutidin und 3- und 4-Ethylpyridin bzw. Mischungen dieser Stoffe in Frage.

Schliesslich sind die Reaktionsbedingungen, unter denen die Hydrocarboxilierung durchgeführt wird, nicht von Bedeutung. Im allgemeinen werden Hydrocarboxilierungsverfahren bei Temperaturen von 80 bis 300, vorzugsweise 150 bis 220° C, und Kohlenmonoxiddrucken von 10 bis 800, vorzugsweise 10 bis 300 bar, durchgeführt. Die Konzentration des als Katalysator eingesetzten Cobalts liegt je nach Art des zur Umsetzung gelangenden Olefins in einem Bereich von 0,005 bis 0,2 G-Atom Cobalt pro Mol Olefin, die Menge des als Cokatalysator verwendeten Pyridins und/oder nichtorthosubstituierten Alkylpyridins in einem Bereich von 3 bis 100, vorzugsweise 5 bis 50 mol pro Grammatom Cobalt und die Menge des pro Mol Olefin eingesetzten Alkanols in einem Bereich von 1 bis 20, vorzugsweise 1 bis 10 mol.

Kritisch für das erfindungsgemässe Verfahren sind jedoch Art und Reihenfolge der einzelnen Verfahrensschritte, die es gestatten, aus den durch Hydrocarboxilierung erhaltenen Carbonsäurealkylestern weitgehend von stickstoffhaltigen Verbindungen befreite Alkohole herzustellen.

Im allgemeinen geht man bei dem erfindungsgemässen Verfahren so vor, dass man die von den übrigen Stoffen des Hydrocarboxilierungsgemisches soweit wie möglich befreiten Carbonsäurealkylester zunächst hydriert. Diese Hydrierung wird unter den für Ester typischen Bedingungen unter Verwendung eines Katalysators, vorzugsweise eines Kupferchromitkatalysators, durchgeführt. Typische Bedingungen für eine Esterhydrierung sind Temperaturen von 150 bis 230, vorzugsweise 180 bis 220° C, und Drucke von 200 bis 400, vorzugsweise 250 bis 350 bar Wasserstoff. Nähere Einzelheiten zu den Reaktionsbedingungen und dem verwendeten Katalysator können der einschlägigen Literatur entnommen werden, z.B.

F. Zymalkowsky, „Katalytische Hydrierung", F. Enke Verlag, Stuttgart, 1965.

Im Anschluss an die Hydrierung erfolgt dann die Behandlung des Alkohols, der in seiner C-Zahl den den Carbonsäurealkylestern zugrundeliegenden Carbonsäuren entspricht, mit dem sauren Ionenaustauscher. Vorzugsweise erfolgt die Behandlung in Gegenwart des der Alkylkomponente der Carbonsäurealkylester entsprechenden Alkohols. Im allgemeinen ist er in einer solche Menge anwesend, die der stöchiometrischen Zusammensetzung der zur Hydrierung eingesetzten Carbonsäurealkylester entspricht. Seine ggf. spätere Abtrennung kann nach bekannten Trennverfahren, wie z.B. Destillation oder Rektifikation, erfolgen. Für das erfindungsgemässe Verfahren geeignete saure Ionenaustauscher sind vorzugsweise Sulfonsäurereste tragende Styrol/Divinylbenzol-Copolymerisate und Carboxylgruppen tragende Copolymerisate von Acrylsäure bzw. Acrylsäurederivaten und Divinylbenzol (nähere Einzelheiten s. „Ullmanns Enzyklopädie der technischen Chemie", Verlag Chemie GmbH, Weinheim/Bergstr. [1977], 4. Aufl., Bd. 13, S. 279 bis 346, „Ionenaustauscher").

Die Behandlung mit saurem Ionenaustauscher kann beispielsweise in einer sogenannten Austauschersäule unter Normalbedingungen (20° C, 1 bar) durchgeführt werden. Abweichungen in der Art und den Bedingungen der Behandlung sind selbstverständlich möglich, sofern sie angebracht sind oder die Stoffeigenschaften des zu reinigenden Gutes sie erfordern.

Mit Hilfe des erfindungsgemässen Verfahrens ist es möglich, Folgeprodukte von durch Hydrocarboxilierung erhaltenen Carbonsäurealkylestern herzustellen, deren auf stickstoffhaltige Verbindungen zurückzuführender Gehalt an elementarem Stickstoff unter 20, vorzugsweise unter 1 Gew.-ppm liegt.

Die nachfolgenden Beispiele dienen der Erläuterung des erfindungsgemässen Verfahrens.

*Vergleichsbeispiel A*

Aus dem Rohprodukt, das durch Umsetzung eines statistischen Isomerengemisches von n-Undecenen, n-Dodecenen und n-Tridecenen unter den Bedingungen, wie sie im Beispiel 1 der DE-OS Nr. 2912489 beschrieben sind, erhalten wurde, wurde durch Rektifikation ein Produkt gewonnen, das hauptsächlich aus den Methylestern der gegenüber den eingesetzten Olefinen um ein Kohlenstoffatom reicheren Carbonsäuren bestand. Bei der Rektifikation verblieben in dem erhaltenen Estergemisch stickstoffhaltige Verbindungen, die kein $\gamma$-Picolin waren und einen analytisch bestimmten Stickstoffgehalt von 147 ppm bedingten. Nach einer Hydrierung der Ester an einem Kupferchromitkatalysator bei 300 atm und 210° C wiesen die bei der Hydrierung überwiegend entstandenen primären Alkohole einen Stickstoffgehalt von 145 ppm auf. Die durch eine sich anschliessende diskontinuierliche Rektifikation über eine Füllkörperkolonne mit ca. 30 theoretischen Trennstufen und bei einem Rücklaufverhältnis von 5 erhaltenen Fraktionen enthielten zwischen 32 und 1450 ppm Stickstoff. Die Fraktionen, die die $C_{12}$- bis $C_{14}$-Alkohole enthielten, hatten einen Durchschnittsgehalt von 118 ppm Stickstoff.

*Vergleichsbeispiel B*

Die Aufarbeitung des Estergemisches gemäss Vergleichsbeispiel A wurde in der Weise durchgeführt, dass das Estergemisch vor der Hydrierung am Kupferchromitkatalysator zunächst bei 25° C über einen handelsüblichen Ionenaustauscher (Lewatit® SPC 118 der Fa. Bayer AG) geleitet wurde. Der Stickstoffgehalt betrug nach der Ionenaustauscherbehandlung 91 ppm, nach der Hydrierung 90 ppm und nach der Rektifikation in den Fraktionen, die die $C_{12}$- bis $C_{14}$-Alkohole enthielten, durchschnittlich 75 ppm.

*Beispiel 1*

Das Estergemisch gemäss Vergleichsbeispiel A wurde nicht vor, sondern nach der Hydrierung unter den im Vergleichsbeispiel A angegebenen Bedingungen mit einem handelsüblichen Ionenaustauscher (Lewatit SPC 118 der Fa. Bayer AG) bei 25° C behandelt. Der Stickstoffgehalt von 147 ppm im Estergemisch und 145 ppm im hydrierten Produkt konnte dadurch auf $< 1$ ppm gesenkt werden. Der durchschnittliche Stickstoffgehalt in den $C_{12}$- bis $C_{14}$-Alkoholfraktionen betrug ebenfalls $< 1$ ppm.

*Beispiel 2*

Beispiel 1 wurde mit dem Unterschied wiederholt, dass anstatt des handelsüblichen Ionenaustauschers Lewatit SPC 118 der handelsübliche Ionenaustauscher Lewatit CNP 80 (ebenfalls Fa. Bayer AG) verwendet wurde. Der durchschnittliche Stickstoffgehalt der $C_{12}$- bis $C_{14}$-Alkoholfraktionen betrug 2 ppm.

## Patentansprüche

1. Verfahren zur Herstellung von weitgehend von stickstoffhaltigen Verbindungen befreiten Alkoholen aus Carbonsäurealkylestern, die durch Umsetzung von Olefinen mit Kohlenmonoxid und Alkanol in Gegenwart eines aus einer Cobaltverbindung und Pyridin und/oder einem nichtorthosubstituierten Alkylpyridin bestehenden Katalysatorsystems bei erhöhtem Druck und erhöhter Temperatur erhalten wurden, dadurch gekennzeichnet, dass man die durch Aufarbeitung des Reaktionsgemisches erhaltenen und dadurch soweit wie möglich von den übrigen Komponenten des Reaktionsgemisches befreiten Carbonsäurealkylester zunächst unter für Ester typischen Bedingungen hydriert und anschliessend eine Behandlung mit einem sauren Ionenaustauscher vornimmt.

2. Verfahren nach Anspruch 1, dadurch gekennzeichnet, dass man die Hydrierung unter Verwendung eines Kupferchromitkatalysators bei Temperaturen von 150 bis 230° C und Drucken von 200 bis 400 bar durchführt.

3. Verfahren nach den Ansprüchen 1 und 2, dadurch gekennzeichnet, dass man als sauren Ionenaustauscher ein sulfoniertes Styrol/Divinylbenzol-Copolymerisat oder carboxylgruppenhaltiges Copolymerisat aus Acrylsäure und/oder ihren Derivaten und Divinylbenzol einsetzt.

4. Verfahren nach den Ansprüchen 1 bis 3, dadurch gekennzeichnet, dass man die Behandlung mit dem sauren Ionenaustauscher in Gegenwart des Alkanols durchführt, das der Alkylkomponente der durch Hydrocarboxilierung hergestellten Carbonsäurealkylester entspricht.

## Claims

1. A process for the production of alcohols which are largely free of nitrogen-containing compounds from alkyl esters of carboxylic acids, which in turn have been obtained by reaction of olefins with carbon monoxide and alkanol at elevated pressure and elevated temperature in the presence of a catalyst system composed of a cobalt compound and pyridine and/or a non-ortho-substituted alkyl pyridine, characterised in that the alkyl carboxylate obtained during working up of the reaction mixture and therefore as far as possible free from the remaining components of the reaction mixture is first hydrogenated under typical conditions for an ester and is then subjected to a treatment with an acidic ion exchanger.

2. A process according to Claim 1, characterised in that the hydrogenation is carried out in the presence of a copper chromite catalyst at temperatures of 150 to 230° C and pressures of 200 to 400 bar.

3. A process according to Claims 1 and 2, characterised in that a sulphonated styrene/divinylbenzene copolymer or carboxyl-group-containing copolymer of acrylic acid and/or its derivatives with divinylbenzene is used as the acidic ion exchanger.

4. A process according to any of Claims 1 to 3, characterised in that the treatment with the acidic ion exchanger is carried out in the presence of the alkanol corresponding to the alkyl moiety of the alkyl carboxylate produced by hydrocarboxylation.

## Revendications

1. Procédé de préparation d'alcools largement débarrassés de composés azotés, en partant d'esters d'alkyle d'acides carboxyliques que l'on a obtenus en faisant réagir des oléfines sur le monoxyde de carbone et un alcanol en présence d'un système catalytique formé d'un composé du cobalt et de pyridine et/ou d'une alkylpyridine non substituée en ortho, à pression élevée et à température élevée, caractérisé par le fait que l'on hydrogène tout d'abord, dans des conditions typiques pour les esters, les esters d'alkyle d'acides carboxyliques obtenus par traitement du mélange réactionnel et, ensuite, débarrassés le plus possible des autres constituants du mélange réactionnel, et en ce qu'on effectue ensuite un traitement par un échangeur d'ions acide.

2. Procédé selon la revendication 1, caracterisé par le fait que l'on effectue l'hydrogénation en utilisant un catalyseur au chromite de cuivre, à des températures de 150 à 230° C et à des pressions de 200 à 400 bar.

3. Procédé selon les revendications 1 et 2, caractérisé par le fait que l'on utilise, comme échangeur d'ions acide, un produit de copolymérisation sulfoné de styrène et de divinylbenzène ou un produit de copolymérisation obtenu en partant d'acide acrylique et/ou de ses dérivés et de divinylbenzène et ·contenant des groupes carboxyle.

4. Procédé selon les revendications 1 à 3, caractérisé par le fait que l'on effectue le traitement par l'échangeur d'ions acide en présence de l'alcanol qui correspond au fragment alkyle de l'ester d'alkyle d'acide carboxylique préparé·par hydrocarboxylation.